# EUROPEAN PATENT APPLICATION

(11) **EP 3 192 653 A1**
(43) Date of publication of application: **19.07.2017**
(21) Application number: 15840800.5
(22) Date of filing: 29.05.2015
(51) Int. Cl.: B32B 15/08, B29C 45/14

(54) **INSERT MOLDED ARTICLE, DEVICE USING SAID INSERT MOLDED ARTICLE, AND METHOD FOR PRODUCING INSERT MOLDED ARTICLE**

(30) Priority: 11.09.2014 JP 2014185345
(71) Applicant: OLYMPUS CORPORATION, Hachioji-shi Tokyo 192-8507 (JP)
(72) Inventor: IMAI, Shigeru, Tokyo 192-8507 (JP); SHIGA, Naohito, Tokyo 192-8507 (JP); ENDO, Tetsuya, Tokyo 192-8507 (JP)
(74) Representative: Schicker, Silvia
(86) International application number: PCT/JP2015/065608
(87) International publication number: WO 2016/038945

(57) **Abstract**

An object is to provide an insert molded article which can maintain high watertightness and has chemical resistance and heat resistance, and a method for producing the same. According to the present invention, there is provided an insert molded article comprising: a first member having electrical conductivity at least at the surface; a surface layer formed on at least a part of the surface of the first member, the surface layer including a compound containing at least one element selected from Si, Ti, Al, Zr, Zn, Cr, Ni, Fe, Mo, B, Be, In, and Sn; and a resin part molded on at least a part of the surface layer such that the resin part adheres tightly to the surface layer.

## Description

### Technical Field

The present invention relates to an insert molded article, an apparatus including the insert molded article, and a method for producing the insert molded article.

### Background Art

Insert molding is a method for producing a molded article by injecting a resin around a core made of a metal. In a conventional insert molded article, a core and a resin are joined depending on only the adhesion thereof or the shrinkage pressure of the resin after being molded. Therefore, joining strength between the core and the resin is insufficient, which makes it difficult to obtain a molded article having watertightness.

For example, Patent Document 1 discloses an injection-molded part wherein at least the outer surface of a core has a metallic anticorrosion layer, a sealing layer is deposited on the anticorrosion layer, and a resin is injected to the outside of the sealing layer. Patent Document 2 discloses a metal-resin composite wherein a chemical conversion treated layer is formed on the surface of a silicon-containing aluminum alloy, and a resin composition containing polyphenylene sulfide or polybutylene terephthalate as a main component is injected onto the surface of the chemical conversion treated layer.

However, these methods also make it difficult to provide a molded article which can have high durability and maintain watertightness over a long period of time. Particularly, parts used for an endoscope are cleaned and sterilized by chemicals such as acids or high temperature and pressure steam. Therefore, in the conventional insert molded article, the resin is deteriorated by the acid or swollen by moisture. This causes a problem that the watertightness cannot be maintained over a long period of time.

Therefore, an insert molded article requiring watertightness cannot be produced by the conventional insert molding process, and thus it is formed by bonding the resin to the core using an adhesive. This causes problems that the work is complicated and requires high cost. This further causes a problem that the durability of the adhesive itself is also low.

### Prior Art Documents

### Patent Documents

Patent Document 1: Jpn. PCT National Publication No. 2009-520610
Patent Document 2: Jpn. Pat. Appln. KOKAI Publication No. 2012-157991

### Summary of Invention

### Technical Problem

It is an object of the present invention to provide an insert molded article which can maintain high watertightness and has chemical resistance and heat resistance, and a method for producing the same.

### Solution to Problem

According to the present invention, there is provided an insert molded article comprising: a first member having electrical conductivity at least at the surface; a surface layer formed on at least a part of the surface of the first member, the surface layer including a compound containing at least one element selected from Si, Ti, Al, Zr, Zn, Cr, Ni, Fe, Mo, B, Be, In, and Sn; and a resin part molded on at least a part of the surface layer such that the resin part adheres tightly to the surface layer.

### Effect of Invention

The present invention can provide an insert molded article which can maintain high watertightness and has chemical resistance and heat resistance, and a method for producing the same. The present invention can also provide a product or apparatus which includes the insert molded article and has watertightness.

### Brief Description of Drawings

FIG. 1 is a schematic partial cross-sectional view of an insert molded article according to one embodiment.
FIG. 2 is a schematic partial cross-sectional view of an insert molded article according to another embodiment.
FIG. 3 is a schematic cross-sectional view of an electric signal connector 10 for endoscopes.
FIG. 4 is a schematic view of a lever according to one embodiment.
FIG. 5 is a schematic view of a switch according to one embodiment.
FIG. 6 is a schematic view of an endoscope tip structural part according to one embodiment.
FIG. 7 is a schematic cross-sectional view of an endoscope tip part according to one embodiment.
FIG. 8 is a schematic view of a connector for connecting an air/water tube according to one embodiment.
FIG. 9 is a schematic view of a watertight packing according to one embodiment.
FIG. 10 is a schematic view of a rigid scope body according to one embodiment.
FIG. 11 is a schematic view of a high-frequency resection apparatus according to one embodiment.

### Description of Embodiments

### (First Embodiment)

An insert molded article according to a first embodiment includes a first member having electrical conductivity at least at the surface; a surface layer formed on at least a part of the surface of the first member, the surface layer including at least one compound containing an element selected from Si, Ti, Al, Zr, Zn, Cr, Ni, Fe, Mo, B, Be, In, and Sn; and a resin part molded on at least a part of the surface layer such that the resin part adheres tightly to the surface layer. Typical examples of the insert molded article include a metal resin composite material.

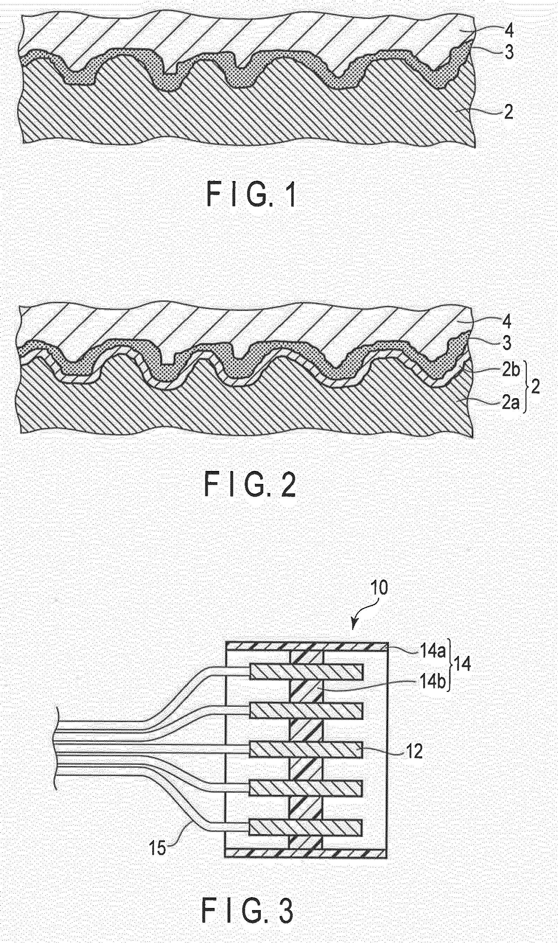

FIG. 1 shows a schematic cross-sectional view showing boundary surfaces among a first member, surface layer, and resin part in an insert molded article according to the present embodiment. The insert molded article includes a surface layer 3 on a surface of a first member 2. The insert molded article includes a resin part 4 provided on the surface layer 3. The insert molded article is produced by using the first member 2, on which the surface layer 3 is previously formed, as a core in insert molding, and injecting a resin onto the surface layer 3 to form the resin part 4.

The first member has electrical conductivity at least at the surface. The first member may be wholly made of an electrically conductive material, for example. Alternatively, the first member may be constituted of an electrically conductive or electrically non-conductive base, and an electrically conductive thin film formed on the surface of the base. The shape of the first member is optional, and depends on a target molded article.

When the first member is wholly made of an electrically conductive material, the first member is made of a material selected from the group consisting of stainless steel, titanium, nickel-titanium (NiTi), silicon steel, cobalt, gold, palladium, nickel, chromium, tungsten, and carbon, for example. These materials may be used alone or as a mixture of two or more. Glass having no electrical conductivity can also be used as the material for the first member. This is considered to be based on the action of ions or the like on the surface of the glass.

FIG. 2 shows a schematic view of a case where the first member includes an electrically conductive thin film on the surface of the base. FIG. 2 is a schematic cross-sectional view showing the boundary surfaces among the first member, surface layer, and resin part in the insert molded article. In FIG. 2, the first member 2 includes an electrically conductive thin film 2b on the surface of a base 2a. In FIG. 2, the insert molded article includes the surface layer 3 on the thin film 2b of the first member 2. The insert molded article further includes the resin part 4 on the surface layer 3.

Examples of a material for the base include copper, stainless steel, titanium, NiTi, silicon steel, cobalt, gold, palladium, nickel, chromium, carbon, glass, sapphire glass, and ceramic (including glass) and resin containing an electrically conductive material in a form of powder or fiber. These may be used alone or as a mixture of two or more. Examples of the resin include polyphenyl sulfone, polysulfone, aromatic polyketone, PEI, polystyrene, polyvinylidene fluoride, polyphenylene sulfide, PPO, polyester, polyurethane, polyimide, polyamide, polyether sulfone, LCP, ABS, and a phenol resin. These may be used alone or as a mixture of two or more.

Examples of the electrically conductive thin film include a metal plating, and a thin film made of a material selected from indium oxide, silicon steel, and sapphire. Examples of the metal plating include a metal plating selected from precious metals such as gold, platinum, and palladium, nickel, titanium, chromium, nickel-titanium, nickel-chromium, nickel-tin, nickel-tin-cobalt, and stainless steel.

The electrically conductive thin film is more preferably gold plating. Since gold has a high transmitting efficiency, gold is suitably used in parts related to electric signals such as an electric signal terminal. Since gold has high durability, gold has advantages of having high rust resistance and high chemical resistance. Since an inert metal plating such as gold plating is apt to be peeled off even if a treated layer is formed on the surface of the inert metal plating, gold plating is not conventionally used in parts requiring watertightness. However, according to the present invention, by forming the surface layer on the gold plating, and further forming the resin part on the surface layer, high watertightness is maintained without deteriorating adhesiveness between the gold plating and the surface layer, which makes it possible to use gold plating.

Next, the surface layer formed on the surface of the first member will be described. The surface layer is a thin layer having electrical conductivity. The thin film having electrical conductivity is, for example, a thin film made of a material having electrical conductivity, a thin film having a structure having electrical conductivity such as a porous structure, or a thin film having an extremely thin thickness to have electrical conductivity. The surface layer is preferably, but is not limited to, a thin film made of a porous body, or a thin film made of a mixed material of a porous body and an organic compound. Since the surface layer has electrical conductivity, the surface layer does not hinder electrical conductivity between the first member and an external member. The surface layer may be formed on the whole surface of the first member, or may be formed on part of the surface. The surface layer may be a single layer or a multi-layer including two or more layers.

Examples of the material for the surface layer include a compound containing an element selected from Si, Ti, Al, Zr, Zn, Cr, Ni, Fe, Mo, B, Be, In, and Sn. Preferably, the compound is an oxide. The compound may be used alone or as a mixture of two or more.

The material for the surface layer is more preferably a silicon compound. Examples of the silicon compound include silicon dioxide, silicon monoxide, and silicon hydroxide.

When the surface layer is the multi-layer, the surface layer preferably includes a layer made of at least one compound selected from a titanium compound, an aluminum compound, and a zirconium compound.

The film thickness of the surface layer is preferably 1 nm or more, and preferably 50 µm or less. The film thickness is more preferably 1000 nm or less, and particularly preferably 100 nm or less. If the film thickness of the surface layer is too large, this may cause cohesive failure.

A preferable density of the surface layer differs according to the material. Preferable densities d20 of the surface layer when the following materials are used will be exemplified later.
Silicon dioxide: 1.1 to 2.2 g/cm³
Titanium oxide: 1.9 to 4.3 g/cm³
Aluminum oxide: 1.9 to 4.1 g/cm³
Zirconium oxide: 2.4 g/cm³
Zinc oxide: 2.8 to 5.6 g/cm³
Chromium oxide (III): 2.6 to 5.2 g/cm³
Nickel oxide: 3.3 to 6.7 g/cm³

Since a too low density causes a decrease in strength, the above range is preferable.

The surface roughness Ra of the surface layer is preferably 0.5 nm to 25 µm. A too low surface roughness does not cause an expected anchor effect, which adversely affects adhesiveness. On the other hand, a too large surface roughness causes an increase in defective sites at the interface between the surface layer and the resin part, which makes it difficult to provide stable adhesiveness.

Next, the resin part will be described. The resin part is formed on at least a part of the surface layer formed on the surface of the first member, and adheres tightly to the surface layer. The shape of the resin part is optional, and depends on a target molded article.

The resin part is preferably made of a polymer material having high durability. Examples of the material for the resin part include a thermoplastic resin, rubber, an elastomer, and a thermosetting resin.

Examples of the thermoplastic resin include a resin selected from PEI, LCP, polyphenyl sulfone, PPO, a polyethylene resin, a polypropylene resin, a polymethyl pentene resin, a polyester resin, a polycarbonate resin, a polyether sulfone resin, an acrylic resin, a polyimide resin, a polysulfone resin, a polystyrene resin, a polyamide resin, a polyphenylene sulfide resin, an ethylene-tetrafluoroethylene copolymer, a polyvinyl fluoride resin, a tetrafluoroethylene-perfluoroether copolymer, a tetrafluoroethylene-hexafluoropropylene copolymer, a polytetrafluoroethylene resin, a polyvinylidene fluoride resin, a polytrifluorochloroethylene resin, aromatic polyketone, and an ethylene-trifluorochloroethylene copolymer. In the aromatic polyketone, a polyether ether ketone resin (PEEK) can be injection-molded and has excellent durability, which is preferable. The polyether ether ketone resin is suitably used in an insert molded article used especially for an endoscope.

Examples of the rubber and elastomer include a resin selected from polyurethane, silicone rubber, fluororubber, natural rubber, neoprene rubber, chloroprene rubber, urethane rubber, acrylic rubber, olefin rubber, styrene-butadiene rubber, acrylonitrile-butadiene rubber, ethylene-propylene rubber, ethylene-propylene-diene rubber, butadiene rubber, butyl rubber, a styrene-based thermoplastic elastomer, and a urethane-based thermoplastic elastomer.

Examples of the thermosetting resin include a resin selected from an epoxy resin, a phenol resin, a cyanate resin, a urea resin, and a guanamine resin.

The materials for the resin part may be used alone or as a mixture of two or more.

In the insert molded article according to the first embodiment described above, the surface layer is present between the first member and the resin part, which is likely to cause the resin part to infiltrate into the fine concavity and convexity of the surface of the first member. As a result, an anchor effect provides strong adhesion between the first member and the resin part. This makes it possible to maintain high watertightness. The insert molded article according to the first embodiment can have more excellent durability such as chemical resistance or heat resistance than that of a conventional molded article formed using an adhesive. Therefore, even if the insert molded article is subjected to cleaning and sterilization of an endoscope using, for example, acid, a chemical substance, and an autoclave with high temperature and pressure steam, the insert molded article can maintain high watertightness over a long period of time.

Furthermore, a sealing process using an adhesive, and a structure for sealing are not required, which can more easily provide a molded article having an excellent quality at low cost.

The insert molded article according to the present embodiment has high chemical resistance and heat resistance, and therefore it is suitably used as parts for endoscopes. Further, the molded article having excellent watertightness and durability can be easily produced by insert molding, and therefore it is suitably used also as parts for various products or apparatuses. Hereinafter, embodiments of the insert molded article or the apparatus including the insert molded article will be exemplified.

FIG. 3 is a schematic cross-sectional view of an electric signal connector 10 for endoscopes. The electric signal connector 10 includes a plurality of electric signal terminals 12 as the first member. Each of the electric signal terminals 12 has a cylindrical shape, and includes a base on which gold plating is applied. Furthermore, a surface layer is formed on the gold plating. The electric signal connector 10 includes, as a resin part, a cylindrically-shaped external cylinder 14a and a disc-shaped electric signal terminal fixing member 14b provided in the external cylinder. The electric signal terminal 12 includes an electric cable 15 at one end.

Thus, the surface layer is present on the electric signal terminal 12, which maintains watertightness between the electric signal terminal 12 and a resin part 14 over a long period of time. Therefore, the electric signal connector 10 can be used as an electric signal connector for endoscopes. Since the durability of a watertight part depends on the durability of not an adhesive but a resin material, the durability of the watertight part can be further improved by using a resin material having high durability. Furthermore, the electric signal connector 10 can be produced by insert molding, which makes it unnecessary to seal the periphery of an electrical contact by an adhesive. Therefore, it can be produced at low cost.

Examples of another embodiment of the insert molded article include a lever and a dial.

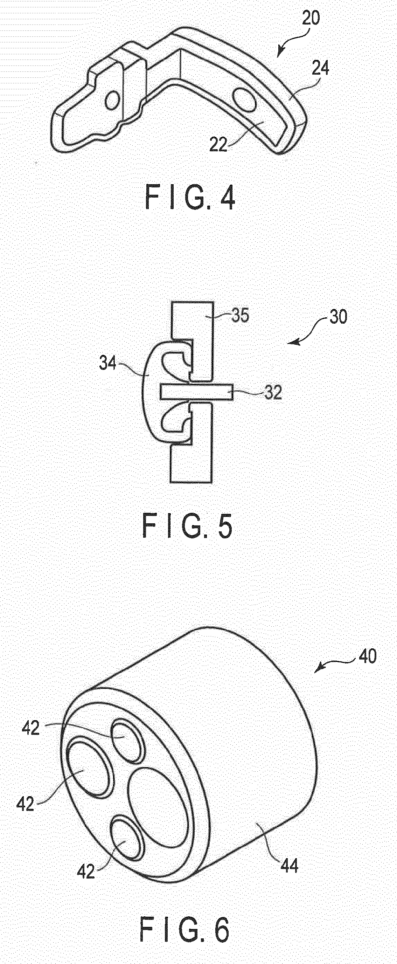

FIG. 4 is a schematic view of a lever 20. In the lever 20, a rust-resistant metal part made of stainless steel or titanium is used as a core 22, and the periphery thereof excluding an inner surface is covered with a resin 24. A surface layer is formed on the surface of the core 22. Thereby, the adhesiveness of the boundary surface between the metal part and the resin can be obtained. Therefore, a molded article having an exposed part of the metal core such as the lever 20 also can prevent water and bacteria or the like from infiltrating from the boundary surface. Such watertightness is not easily degraded by processes of cleaning, disinfection, and sterilization for endoscopes, thus adhesiveness can be maintained.

Examples of another embodiment of the insert molded article include a switch.

FIG. 5 shows a schematic view of a switch 30. The switch 30 includes a metal switch action part 32 as a core, and includes an operating part 34 made of an elastomer on an outer surface of the article. The integrated switch action part 32 and operating part 34 are installed on a fixing member 35. A surface layer is formed on the surface of the switch action part 32. Since this firmly joins the operating part 34 made of an elastomer and the metal switch action part 32, the joining of the operating part 34 and switch action part 32 is not easily degraded even when the operating part 34 is pressed and deformed. Therefore, a more durable part can be inexpensively provided.

Examples of another embodiment of the insert molded article include an endoscope tip structural part (tip frame).

FIG. 6 is a schematic view of an endoscope tip structural part 40. The endoscope tip structural part 40 includes glass parts 42 as a tip optical component. The glass parts 42 are used as a core, and the resin part 44 is formed around the core. A surface layer is formed on the surface of the glass parts 42. Thereby, the glass parts 42 and the resin part 44 are firmly joined, which maintains watertightness on the boundary surface. This can prevent water and bacteria or the like from infiltrating from the boundary surface. Such watertightness is not easily degraded by processes of cleaning, disinfection, and sterilization for endoscopes, thus adhesiveness can be maintained. The watertightness can prevent the boundary between the parts from being polluted by bacteria, which leads to the provision of a safe endoscope.

Examples of another embodiment of the insert molded article include air/water tube line systems for endoscopes.

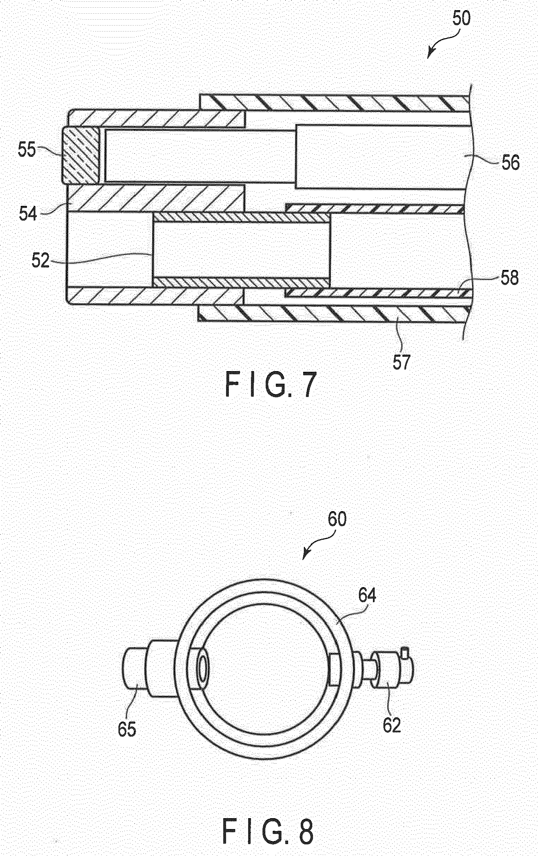

FIG. 7 shows a schematic cross-sectional view of an endoscope tip part 50. In FIG. 7, a tip structural part 54 for endoscopes, an optical transmission tube 56, and an air/water tube 58 are disposed in an endoscope inserting part 57.

The air/water tube 58 is a metal tube for sending or suctioning air, water, or a chemical or the like. As a core, a rust-resistant tubing part 52 made of stainless steel, titanium or the like is used. One end of the core is joined to the tip structural part 54, and the other end thereof is joined to the air/water tube 58. A surface layer is formed on the surface of the tubing part 52. This maintains watertightness on the boundary surface between the tubing part 52 and the tip structural part 54 or the air/water tube 58, which can prevent water and bacteria or the like from infiltrating from the boundary surface. Such watertightness is not easily degraded by processes of cleaning, disinfection, and sterilization for endoscopes, thus adhesiveness can be maintained. The watertightness can prevent the boundary between the parts from being polluted by bacteria, which leads to the provision of a safe endoscope.

Even if a pressure difference between the air/water tube 58 pressure and ambient pressure occurs when the air/water tube 58 sends or suctions air, water, or a chemical or the like, watertightness is maintained on the bonded surface of the air/water tube 58 and resin, which causes no infiltration of water or a chemical from the inside of the air/water tube 58 to the outside thereof, i.e., into the endoscope.

FIG. 8 is a schematic view of a connector for connecting an air/water tube or the like. As a core, rust-resistant connecting parts 62 and 65 made of stainless steel, titanium or the like are used, and one end of each of the connecting parts is located in a resin part 64. A surface layer is formed on the surfaces of the connecting parts 62 and 65. This maintains watertightness on the boundary surface between the connecting parts 62, 65 and the resin part 64, which can prevent water and bacteria or the like from infiltrating from the boundary surface.

Examples of another embodiment of the insert molded article include a watertight packing.

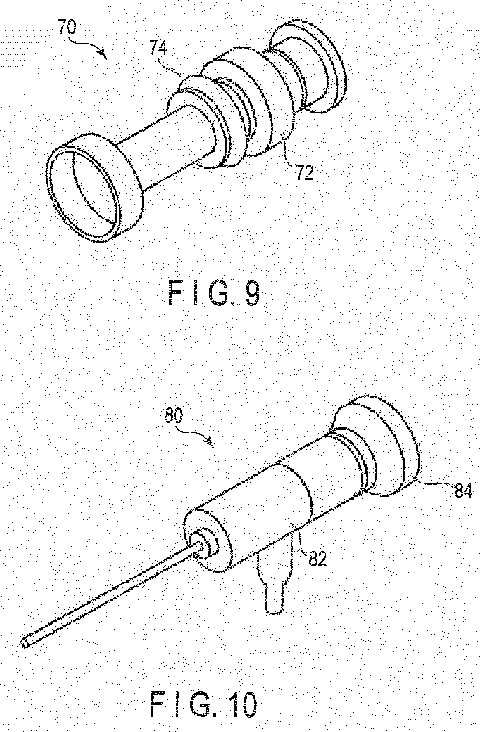

FIG. 9 is a schematic view of a watertight packing 70. A main body 72 made of a rust-resistant metal such as stainless steel or titanium is used as a core. A part of the core includes a watertight packing 74 made of an elastomeric material. A surface layer is formed on the surface of the packing 74. Thereby, the adhesiveness of the boundary surface between the metal and resin can be obtained. Therefore, watertightness can be secured. The resin can be directly insert-molded on a rust-resistant material, which makes it possible to mold a packing having a free structure and shape according to the structure and shape of the part.

Examples of another embodiment of the insert molded article include an eye lens cover for medical rigid endoscopes.

FIG. 10 shows a schematic view of a rigid scope main body 80. A part made of a rust-resistant metal such as stainless steel or titanium is used as a core 82. The rigid scope main body 80 includes an eye lens cover 84 covering the periphery of one end of the core 82. The eye lens cover 84 disposed as an outer covering of the core 82 serves as an insulator preventing a medical worker from being electrocuted when an electrosurgical knife or the like is used. A surface layer is formed on the surface of the core 82. This maintains watertightness on the boundary surface between the core 82 and the eye lens cover, which can prevent water and bacteria or the like from infiltrating from the boundary surface. Such watertightness is not easily degraded by processes of cleaning, disinfection, and sterilization for endoscopes, thus adhesiveness can be maintained.

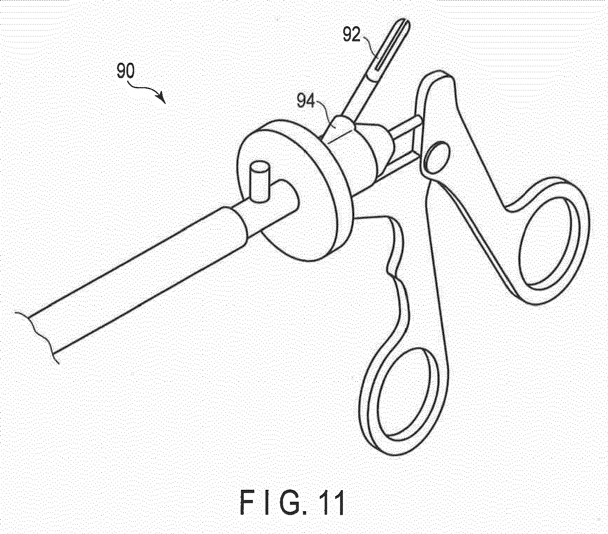

Examples of another embodiment of the insert molded article include an exterior part of a medical therapeutic apparatus. Examples of the exterior part include a shaft and a handle. Examples of the therapeutic apparatus include an ultrasonic coagulation and cutting apparatus, a high-frequency resection apparatus, and an electrosurgical knife. FIG. 11 shows a schematic view of a high-frequency resection apparatus 90. A metal part having rust-resistant performance such as stainless steel or titanium is used as a core 92, and the periphery of one end of the core 92 is covered with a resin part 94. The resin part 94 serves as an insulator preventing a medical worker from being electrocuted. A surface layer is formed on the surface of the core 92. This maintains watertightness on the boundary surface between the core 92 and the resin part 94, which can prevent water and bacteria or the like from infiltrating from the boundary surface. Such watertightness is not easily degraded by processes of cleaning, disinfection, and sterilization for endoscopes, thus adhesiveness can be maintained.

Examples of another embodiment of the insert molded article include a waterproof apparatus. Examples of the waterproof apparatus include communication terminals such as a waterproof smart phone or tablet, and medical communication terminals. A waterproof product can be easily produced by using the insert molded article of the present embodiment for an electrical contact for charging these terminals.

In these apparatuses, for example, there can be used a core in which an electrically conductive thin film is formed on the surface of a metal having excellent electric conductivity such as copper and phosphor bronze. Examples of the electrically conductive thin film include a plating made of a precious metal such as gold or palladium or an inert metal having excellent electric conductivity; or a surface coating provided by sputtering.

Examples of another embodiment of the insert molded article include an electric pot, an electric toothbrush, a waterproof camera, and a gear and shaft which are obtained by molding a gear part by insert molding around a metal shaft. Required strength can be provided simply by insert molding without the need for bonding the gear and a resin using an adhesive. Therefore, the insert molded article can be produced easily and inexpensively.

Examples of another embodiment of the insert molded article include an injector. The injector can be produced by forming a surface layer on the surface of an injection needle and molding a flange portion around the injection needle by insert molding. The injector thus produced has watertightness between the injection needle and the flange portion. Since the watertightness is not degraded by pressure during injection, the watertight part is not affected by various chemicals, and thus can prevent the outflow and contamination of the chemicals contained therein.

### (Second Embodiment)

A method for producing the insert molded article according to the first embodiment will be described. The producing method includes: forming a surface layer including a compound containing at least one element selected from Si, Ti, Al, Zr, Zn, Cr, Ni, Fe, Mo, B, Be, In, and Sn on a surface of a first member having electrical conductivity at least at the surface; installing the surface layer-formed first member in a molding die; and injecting a molten resin into the molding die to mold the resin on the surface layer formed on the surface of the first member.

Forming the surface layer can utilize a sputtering method, an electron beam vapor deposition method, an ion plating method, a chemical vapor deposition (CVD) method, a pyrosol method, a spraying method, and a dip method or the like without limitation. Among these, CVD is preferred, and thermal CVD is more preferred. Thermal CVD has advantages of facilitating the formation of a thin film and providing a high film formation rate and large treated area with respect to an apparatus scale.

In one embodiment, formation of a surface layer utilizes spraying a solution of a compound containing at least one element selected from Si, Ti, Al, Zr, Zn, Cr, Ni, Fe, Mo, B, Be, In, and Sn into a thermal CVD flame.

In another embodiment, formation of a surface layer utilizes spraying a fuel gas flame containing a compound containing at least one element selected from Si, Ti, Al, Zr, Zn, Cr, Ni, Fe, Mo, B, Be, In, and Sn to a part of or the whole of the surface of the first member.

When the surface layer is made of a Si oxide, the surface layer constituted of a porous silica film can be formed by applying an alkali metal silicate salt to the surface of the first member, and carbonating the alkali metal silicate salt.

In another embodiment, the surface layer can be formed as follows. First, a metal oxide precursor is hydrolyzed and polymerized to generate a sol. A first member is immersed into the sol. The sol is energized with the first member as a negative electrode in this state. Then, the first member is pulled up, and then heated. Thereby, a gel layer made of the metal oxide is formed on the surface of the first member. The surface layer can be formed by heat-treating the gel layer so that the gel layer has a predetermined density.

In another embodiment, the surface layer can be formed as follows. First, a surfactant is dissolved in an aqueous solution so that its concentration becomes equal to or more than a critical micelle concentration, thereby causing micelle particles to be formed. The solution is then left to stand until the micelle particles form a packing structure and convert to colloidal crystals. Then, a silica source such as tetraethoxysilane is added to the solution, and a very small amount of acid or base is further added as a catalyst. This allows a sol-gel reaction to proceed in gaps between the colloidal crystals, to form a silica gel skeleton. The obtained solution is applied to the surface of the first member, and fired at a high temperature to degrade and remove the surfactant, whereby a pure mesoporous silica layer can be formed.

### Examples

### <Example 1>

A first member made of phosphor bronze to which a Ni-Au plating was applied was used as a test piece. Fuel gas containing an alkylsilane compound was sprayed to the test piece for 0.5 seconds to form a porous silica film on the surface of the test piece. The formed surface layer had a film thickness of 100 nm, a density d20 of 2.1 g/cm³, and surface roughness Ra of 30 nm. The thickness of the surface layer was calculated from a depth profile via XPS. The density was calculated by dividing the increased weight of the test piece after the formation of the surface layer by the volume of the surface layer obtained from the thickness of the surface layer. The surface roughness was measured with an atomic force microscope.

Injection molding was performed using the test piece on which the surface layer was formed, and a resin. An insert molded article of Example 1-1 was produced using polyphenyl sulfone (PPSU) manufactured by Solvay as the resin. An insert molded article of Example 1-2 was produced using PEEK manufactured by Victrex as the resin.

### <Example 2>

A first member made of phosphor bronze to which a Ni-Au plating was applied was used as a test piece. The test piece was immersed into an aqueous solution of an alkali metal silicate. Then, the test piece, carbon acid gas, and water were placed into an airtight container, and the test piece was subjected to a carbonation treatment while a constant temperature and humidity were maintained. By this treatment, a porous silica film was formed on the surface of the test piece. The formed surface layer had a film thickness of 10 µm, a density d20 of 2.0 g/cm³, and surface roughness Ra of 50 nm.

Injection molding was performed using the test piece on which the surface layer was formed, and a resin. An insert molded article of Example 2-1 was produced using PPSU as the resin. An insert molded article of Example 2-2 was produced using PEEK as the resin.

### <Example 3>

A first member made of phosphor bronze to which a Ni-Au plating was applied was used as a test piece. A mixed solution obtained by mixing 100 g of tetraethoxysilane, 0.16 g of nitric acid, 5.4 g of water, and 80 ml of isopropyl alcohol was heated at 90°C for 2 hours while being refluxed, to prepare a sol. The test piece was immersed into the sol while the temperature of the sol was maintained at 25±5°C. Energization was performed at 10 V for 10 minutes using the test piece as a negative electrode and a platinum-coated titanium electrode as a positive electrode. Thereby, a gel layer was electrodeposited on the surface of the test piece. The test piece was pulled up, and heat-treated at 80°C for 20 minutes, and at 150°C for 30 minutes to form a surface layer made of amorphous silica. The surface layer had a film thickness of 1 µm, a density d20 of 2.2 g/cm³, and surface roughness Ra of 40 nm.

Injection molding was performed using the test piece on which the surface layer was formed, and a resin. An insert molded article of Example 3-1 was produced using PPSU as the resin. An insert molded article of Example 3-2 was produced using PEEK as the resin.

### <Comparative Example 1>

A first member made of phosphor bronze to which a Ni-Au plating was applied was used as a test piece. By an electrochemical treatment, a layer made of a triazinethiol compound (RTD) was formed on the surface of the test piece.

Injection molding was performed using the test piece on which the RTD layer was formed, and a resin. An insert molded article of Comparative Example 1-1 was produced using PPSU as the resin. An insert molded article of Comparative Example 1-2 was produced using PEEK as the resin.

### <Comparative Example 2>

A first member made of phosphor bronze to which a Ni-Au plating was applied was used as a test piece. The test piece was subjected to a silane coupling treatment using an aminosilane type silane coupling agent (KBP43) manufactured by Shin-Etsu Chemical Co., Ltd. to form a treated layer.

Injection molding was performed using the test piece on which the treated layer was formed, and a resin. An insert molded article of Comparative Example 2-1 was produced using PPSU as the resin. An insert molded article of Comparative Example 2-2 was produced using PEEK as the resin.

### <Comparative Example 3>

A first member made of phosphor bronze to which a Ni-Au plating was applied was used as a test piece. Injection molding was performed using the test piece and a resin. An insert molded article of Comparative Example 3-1 was produced using PPSU as the resin. An insert molded article of Comparative Example 3-2 was produced using PEEK as the resin.

### <Evaluation>

Tests for evaluating strength and watertightness were performed using the insert molded article of each of Examples and Comparative Examples. For the evaluation of the strength, a tensile test was performed using an autograph manufactured by Shimadzu Corporation, and the adhesion strength of the interface between the test piece and the resin was measured. The strength of each of Examples and Comparative Examples was shown as a relative evaluation when the strength of Comparative Example 3-1 or 3-2 was defined as 1.

For the evaluation of watertightness, an air leak test was carried out in water, and air bubbles generated from the interface between the test piece and the resin were observed. A case where the air bubbles were not generated was mentioned as good, and a case where the air bubbles were generated was mentioned as poor.

Furthermore, the insert molded article of each of Examples and Comparative Examples was sterilized using hydrogen peroxide gas, and the same tests were performed after the sterilization.

The above results are shown in Table 1.

**[Table 1]**

| | | Before hydrogen peroxide sterilization | | After hydrogen peroxide sterilization | |
|---|---|---|---|---|---|
| | Resin | Strength | Watertightness | Strength | Watertightness |
| Example 1-1 | PPSU | 4 | Good | 2 | Good |
| Example 2-1 | PPSU | 3 | Good | 2 | Good |
| Example 3-1 | PPSU | 4 | Good | 2 | Good |
| Comparative Example 1-1 | PPSU | 2 | Poor | 1 | Poor |
| Comparative Example 2-1 | PPSU | 2 | Poor | 1 | Poor |
| Comparative Example 3-1 | PPSU | 1 | Poor | 1 | Poor |
| Example 1-2 | PEEK | 6 | Good | 6 | Good |
| Example 2-2 | PEEK | 2 | Good | 2 | Good |
| Example 3-2 | PEEK | 3 | Good | 3 | Good |
| Comparative Example 1-2 | PEEK | 2 | Poor | 2 | Poor |
| Comparative Example 2-2 | PEEK | 2 | Poor | 2 | Poor |
| Comparative Example 3-2 | PEEK | 1 | Poor | 1 | Poor |

Table 1 shows that Examples 1 to 3 had high strength and had watertightness. The strength and the watertightness were shown to be maintained after the sterilization using hydrogen peroxide.

### Reference Signs List

- 2: first member
- 2a: base
- 2b: thin film
- 3: surface layer
- 4: resin part
- 10: electric signal connector
- 12: electric signal terminal
- 14: resin part
- 14a: external cylinder
- 14b: electric signal terminal-fixing member
- 15: electric cable

## Claims

1. An insert molded article comprising:
a first member having electrical conductivity at least at the surface;
a surface layer formed on at least a part of the surface of the first member, the surface layer including a compound containing at least one element selected from Si, Ti, Al, Zr, Zn, Cr, Ni, Fe, Mo, B, Be, In, and Sn; and
a resin part molded on at least a part of the surface layer such that the resin part adheres tightly to the surface layer.

2. The insert molded article according to claim 1, wherein the first member is made of a material selected from the group consisting of stainless steel, titanium, nickel-titanium (NiTi), silicon steel, cobalt, gold, palladium, nickel, chromium, tungsten, carbon, and combinations thereof.

3. The insert molded article according to claim 1, wherein the first member includes:
a base; and
a thin film having electrical conductivity, formed on a surface of the base.

4. The insert molded article according to claim 3, wherein the thin film is at least one thin film selected from the group consisting of a metal plating selected from gold, platinum, palladium, nickel, titanium, chromium, nickel-titanium, nickel-chromium, nickel-tin, nickel-tin-cobalt, and stainless steel; an indium oxide thin film; a silicon steel thin film; and a sapphire thin film.

5. The insert molded article according to any one of claims 1 to 4, wherein the resin part is made of at least one resin selected from the group consisting of PEI, LCP, polyphenyl sulfone, PPO, a polyethylene resin, a polypropylene resin, a polymethyl pentene resin, a polyester resin, a polycarbonate resin, a polyether sulfone resin, an acrylic resin, a polyimide resin, a polysulfone resin, a polystyrene resin, a polyamide resin, a polyphenylene sulfide resin, an ethylene-tetrafluoroethylene copolymer, a polyvinyl fluoride resin, a tetrafluoroethylene-perfluoro ether copolymer, a tetrafluoroethylene-hexafluoropropylene copolymer, a polytetrafluoroethylene resin, a polyvinylidene fluoride resin, a polytrifluorochloroethylene resin, aromatic polyketone, and an ethylene-trifluorochloroethylene copolymer.

6. The insert molded article according to any one of claims 1 to 4, wherein the resin part is made of at least one resin selected from the group consisting of polyurethane, silicone rubber, fluororubber, natural rubber, neoprene rubber, chloroprene rubber, urethane rubber, acrylic rubber, olefin rubber, styrene-butadiene rubber, acrylonitrile-butadiene rubber, ethylene-propylene rubber, ethylene-propylene-diene rubber, butadiene rubber, butyl rubber, a styrene-based thermoplastic elastomer, and a urethane-based thermoplastic elastomer.

7. The insert molded article according to any one of claims 1 to 4, wherein the resin part is made of at least one resin selected from the group consisting of an epoxy resin, a phenol resin, a cyanate resin, a urea resin, and a guanamine resin.

8. The insert molded article according to any one of claims 1 to 7, wherein the first member is a cylindrical electric signal terminal.

9. An electric signal connector comprising the insert molded article according to any one of claims 1 to 7.

10. An endoscope comprising the insert molded article according to any one of claims 1 to 7.

11. A method for producing an insert molded article comprising:
forming a surface layer including a compound containing at least one element selected from Si, Ti, Al, Zr, Zn, Cr, Ni, Fe, Mo, B, Be, In, and Sn on a surface of a first member having electrical conductivity at least at the surface;
installing the surface layer-formed first member in a molding die; and
injecting a molten resin into the molding die to mold the resin on the surface layer formed on the surface of the first member.

12. The method according to claim 11, wherein the forming the surface layer is spraying a fuel gas flame containing a compound containing at least one element selected from Si, Ti, Al, Zr, Zn, Cr, Ni, Fe, Mo, B, Be, In, and Sn onto the surface of the first member.
